# EUROPEAN PATENT APPLICATION

(11) **EP 4 403 916 A1**
(43) Date of publication of application: **24.07.2024**
(21) Application number: 23156433.7
(22) Date of filing: 14.02.2023
(51) Int. Cl.: G01N 33/533, G01N 33/543, G01N 33/577

(54) **BDNF QUANTITATIVE IMMUNOCHROMATOGRAPHIC TEST STRIP AND PREPARATION METHOD THEREOF**

(30) Priority: 18.01.2023 US 202318098193
(71) Applicant: Anbio Biotechnology Limited, Road Town VG1110 Tortola (VG); Wang, Jincheng, Xiamen City Fujian (CN)
(72) Inventor: WANG, JINCHENG, Xiamen City, Fujian (CN); WANG, DAMING, Xiamen City (CN)
(74) Representative: Sach, Greg Robert

(57) **Abstract**

A BDNF quantitative immunochromatographic test strip and its preparation method, comprises: a substrate and a coated film, on which a sample pad, a coated line area and a water-absorbing pad are arranged; the coated line area comprises a marking line, a detecting line and quality control line, which are arranged in parallel and separated from each other; the marking line is arranged close to the sample pad, the detecting line is arranged between the marking line and the quality control line, the quality control line is arranged close to the water-absorbing pad; the marking line is the marking line coated with fluorescent microspheres marked with first BDNF monoclonal antibody and quality control antigen or antibody, the quality control line is the quality control line coated with quality control antibody or antigen, and the detection line is the detection line coated with second BDNF monoclonal antibody.

## Description

### TECHNICAL FIELD

The present application relates to the technical field of in vitro diagnostic reagents, specifically a BDNF quantitative immunochromatographic test strip and a preparation method thereof.

### BACKGROUND

The clinical detection of depression mostly adopts the diagnosis of symptomology, which relies heavily on the subjective judgment of clinical doctors, and lacks suitable objective indicators. There is a correlation between the level of brain-derived nerve growth factor and the severity of depression. The existing detection technology of this project is detection by enzyme-linked immunoassay (ELISA). The content of brain-derived nerve growth factor in the sample is detected by the coated antibody and the enzyme-marked antibody on the microwell plate, it comprises the steps of adding sample-incubating-washing- adding enzyme -second incubating-second washing-adding substrate-third incubating-termination and detection.

Components such as microwell plates and enzyme-marked solutions used in ELISA reagents need to be refrigerated. At the same time, in order to maintain the detection specificity, a step-by-step reaction is required, so multiple incubations and washings are required. This detection method has the following problems, that is, the reaction time is long (reaction time 90-110 minutes), there are many operation steps, and many instruments and equipment (incubator, plate washer, microplate reader, etc.) are required, it takes up a lot of laboratory space, and the reagents need to be refrigerated (2-8°C) for transportation and storage, reagents have a short validity period (6 months to 12 months), etc.

In view of this, it is an urgent problem to be solved in this technical field to overcome the shortcomings of the products in the prior art.

### SUMMARY OF THE APPLICATION

The technical problem mainly solved by the present application is to provide a BDNF quantitative immunochromatographic test strip and its preparation method. The present application adopts the principle of fluorescence immunochromatography for detection, the reagent components are dry components, and the reaction process is spontaneously completed by liquid automatic chromatography, which can overcome many problems existing in the original technology.

In order to solve the above-mentioned technical problems, a technical solution provided by the present application is: A BDNF quantitative immunochromatographic test strip comprises: a substrate and a coated film arranged on the substrate, wherein a sample pad, a coated line area and a water-absorbing pad are arranged on the coated film; the coated line area comprises a marking line, a detecting line and quality control line, wherein the marking line, the detecting line and the quality control line are arranged in parallel and separated from each other; the marking line is arranged close to the sample pad, the detecting line is arranged between the marking line and the quality control line, and the quality control line is arranged close to the water-absorbing pad;

wherein the marking line is the marking line coated with fluorescent microspheres marked with first BDNF monoclonal antibody and quality control antigen or antibody, the quality control line is the quality control line coated with quality control antibody or antigen, and the detection line is the detection line coated with second BDNF monoclonal antibody that can specifically bind to the antigen to be tested BDNF, and the first BDNF monoclonal antibody is different from the second BDNF monoclonal antibody.

Further, the antibody marked with fluorescent microspheres on the marking line is diluted with a marking buffer, and the marking buffer comprises 0.2%~3% BSA, 5%-20% sucrose, 0.1%-0.5% Tween-20, 0.05-0.1% Proclin300, a balance of 0.01-0.05M, and PBS with a pH of 7.4.

Further, the antibodies coated on the quality control line and the detection line are all diluted with a coating buffer, the coating buffer comprises 5% to 20% sucrose, a balance of 0.01-0.05M, and PBS with a pH of 7.4.

Further, the coating film is a nitrocellulose film, and the climbing speed of the nitrocellulose film is 95 s - 180 s/4 cm.

Further, the concentration of the first BDNF monoclonal antibody marked with fluorescent microspheres is 0.5-2 mg/ml, and a dosage on the test strip is 0.03-0.3 µg/cm2, diluted at a ratio of 10%-40%; the concentration of the quality control antigen or antibody marked with the fluorescent microsphere is 0.5-2 mg/ml, diluted at a ratio of 5%-20%, a dosage on the test strip is 0.005-0.1 µg/cm².

Further, the concentration of the quality control antibody or antigen coated on the quality control line is 0.5 - 2 mg/ml, and a dosage is 0.5-1.5 µg/cm; the concentration of the second BDNF monoclonal antibody coated on the detecting line is 0 .5~2 mg/ml, the dosage is 0.1-1 µg/cm.

Further, a distance between the marking line, the detecting line and the quality control line is 2-5 mm.

Further, a diameter of the fluorescent microsphere is 0.1 µm-1 µm, and an emission wavelength after being excited is 420 nm-800 nm.

In order to solve the above technical problems, a technical solution provided by the present application is to provide a preparation method for a BDNF quantitative immunochromatographic test strip, wherein the preparation method is used to prepare the BDNF quantitative immunochromatography test strip, and the preparation method comprises:

overlap a water-absorbing pad and a coating film on a substrate;

dilute fluorescent microspheres marked with first BDNF monoclonal antibody with marking buffer and dilute fluorescent microspheres marked with quality control antigen or antibody with marking buffer, mix the two marked microspheres, and spray on a binding pad to form a marker binding pad;

dilute the quality control antigen or antibody and second BDNF monoclonal antibody that can specifically bind to the antigen to be tested BDNF with coating buffer, and draw the diluted two raw materials in parallel in order to form a detecting line and a quality control line on the coating film;

paste the marker binding pad and the sample pad on the substrate in an overlapping manner.

Further, the overlap a water-absorbing pad and a coating film on a substrate comprising:

overlap the water-absorbing pad and the coated film on the substrate, and one end of the water-absorbing pad is close to the edge of the substrate, and one side of the coated film should be overlapped by the water-absorbing pad for 1-2 mm.

The beneficial effects of the present application are: the present application provides a BDNF quantitative immunochromatographic test strip and a preparation method thereof, and the BDNF quantitative immunochromatographic test strip comprises a substrate and a coated film arranged on the substrate, wherein a sample pad, a coated line area and a water-absorbing pad are arranged on the coated film; the coated line area comprises a marking line, a detecting line and quality control line, wherein the marking line, the detecting line and the quality control line are arranged in parallel and separated from each other; the marking line is arranged close to the sample pad, the detecting line is arranged between the marking line and the quality control line, and the quality control line is arranged close to the water-absorbing pad; wherein the marking line is the marking line coated with fluorescent microspheres marked with first BDNF monoclonal antibody and quality control antigen or antibody, the quality control line is the quality control line coated with quality control antibody or antigen, and the detection line is the detection line coated with second BDNF monoclonal antibody that can specifically bind to the antigen to be tested BDNF, and the first BDNF monoclonal antibody is different from the second BDNF monoclonal antibody.

The present application adopts the principle of fluorescence immunochromatography for detection, the reagent components are dry components, and the reaction process is spontaneously completed by liquid automatic chromatography, which can overcome many problems existing in the original technology. Using fluorescent immunochromatographic reagents to detect the content of brain-derived nerve growth factor in human samples to assist in the diagnosis of depression.

The present application adopts the principle of fluorescence immunochromatography, which has a short reaction time (15 minutes to complete), simple operation steps (one-step reaction after adding a sample, no need for washing and multiple incubations), and requires less equipment (only one fluorescence analyzer is needed), the reagents are transported at room temperature (2-30°C), and the validity period is long (18 months).

### BRIEF DESCRIPTION OF THE DRAWINGS

In order to illustrate the technical solutions of the embodiments of the present application more clearly, the following briefly introduces the drawings that are used in the embodiments of the present application. Apparently, the drawings described below are only some embodiments of the present application, and those skilled in the art can also obtain other drawings based on these drawings without creative efforts.
Fig. 1 is a structural diagram of the BDNF quantitative immunochromatography test strip according to the embodiment of the present application;
Fig. 2 is a schematic flow chart of the preparation method of the BDNF quantitative immunochromatography test strip according to the embodiment of the present application;
Fig. 3 is a schematic structural diagram of a detection card provided by an embodiment of the present application.

### DETAILED DESCRIPTION

The following will clearly and completely describe the technical solutions in the embodiments of the present application with reference to the drawings in the embodiments of the present application. Apparently, the described embodiments are only some of the embodiments of the present application, not all of them. Based on the embodiments in the present application, all other embodiments obtained by those skilled in the art without making creative efforts belong to the protection scope of the present application.

In the description of the present application, it should be understood that the orientation or positional relationship indicated by the terms "center", "longitudinal", "transverse", "length", "width", "thickness", "upper", "lower", "front", "rear", "left", "right", "vertical", "horizontal", "top", "bottom", "inner", "outer", etc. is based on the orientation or positional relationship shown in the drawings, and is only for the convenience of describing the present application and simplifying the description. It is not intended to indicate or imply that the device or element referred to must have a particular orientation, be constructed, or operate in a particular orientation, and thus should not be construed as limiting the application. In addition, the terms "first" and "second" are used for descriptive purposes only, and cannot be interpreted as indicating or implying relative importance or implicitly specifying the quantity of indicated technical features. Thus, features defined as "first" and "second" may explicitly or implicitly include one or more features. In the description of the present application, "plurality" means two or more, unless otherwise specifically defined.

In the present application, the word "exemplary" is used to mean "serving as an example, illustration or description". Any embodiment described in the present application as "exemplary" is not necessarily to be construed as preferred or advantageous over other embodiments. The following description is given to enable any person skilled in the art to make and use the present application. In the following description, details are set forth for purposes of explanation. It should be understood that a person skilled in the art would recognize that the present application may be practiced without these specific details. In other instances, well-known structures and processes are not described in detail to avoid obscuring the description of the present application with unnecessary detail. Thus, the present application is not intended to be limited to the embodiments shown, but is to be accorded the widest scope consistent with the principles and features disclosed in the present application.

### Embodiment 1

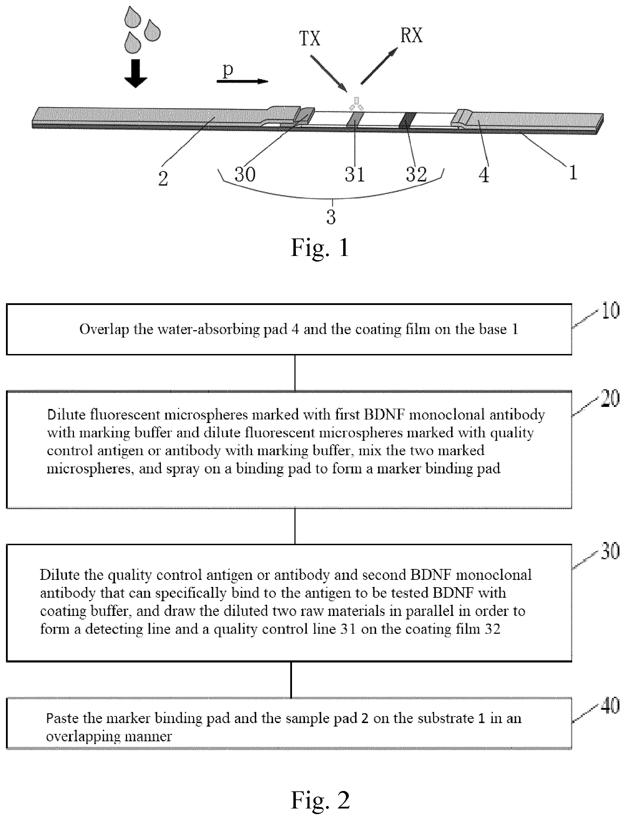

Referring to Fig. 1, the present embodiment provides a BDNF quantitative immunochromatographic test strip, comprises a substrate 1 and a coated film (not labeled) arranged on the substrate 1, wherein a sample pad 2, a coated line area 3 and a water-absorbing pad 4 are arranged on the coated film; the coated line area 3 comprises a marking line 30, a detecting line 31 and quality control line 32, wherein the marking line 30, the detecting line 31 and the quality control line 32 are arranged in parallel and separated from each other; the marking line 30 is arranged close to the sample pad 2, the detecting line 31 is arranged between the marking line 30 and the quality control line 32, and the quality control line 32 is arranged close to the water-absorbing pad 4;

Wherein the marking line 30 is the marking line 30 coated with fluorescent microspheres marked with first BDNF(Brain-derived Neurotrophic Factor) monoclonal antibody and quality control antigen or antibody, the quality control line 32 is the quality control line 32 coated with quality control antibody or antigen, and the detection line 31 is the detection line 31 coated with second BDNF monoclonal antibody that can specifically bind to the antigen to be tested BDNF, and the first BDNF monoclonal antibody is different from the second BDNF monoclonal antibody.

The BDNF to be tested is human BDNF, and BDNF is the most abundant neurotrophic factor in the body.

The marking line 30 is arranged on the marker binding pad, and the marking line 30 is prepared on the marker binding pad, wherein the marker binding pad and the sample pad 2 overlap on the coating film, and the sample pad 2 overlaps on the marker binding pad.

The sample pad 2 is used to collect the BDNF to be tested, and perform chromatography along the arrow p, where the arrow p is the chromatography direction, and the sample passes through the marking line 30, the detection line 31 and the quality control line 32 in sequence.

In one of the embodiments, the antibody marked with fluorescent microspheres on the marking line 30 is diluted with a marking buffer, and the marking buffer comprises 0.2%~3% BSA, 5%-20% sucrose, 0.1%-0.5% Tween-20, 0.05-0.1% Proclin300, a balance of 0.01-0.05M, and PBS with a pH of 7.4.

In one of the embodiments, the antibodies coated on the quality control line and the detection line are all diluted with a coating buffer, the coating buffer comprises 5% to 20% sucrose, a balance of 0.01-0.05M, and PBS with a pH of 7.4.

In one of the embodiments, the coating film is a nitrocellulose film, and the climbing speed of the nitrocellulose film is 95 s - 180 s/4 cm.

In one of the embodiments, the concentration of the first BDNF monoclonal antibody marked with fluorescent microspheres is 0.5-2 mg/ml, and a dosage on the test strip is 0.03-0.3 µg/cm2, diluted at a ratio of 10%-40%; the concentration of the fluorescent microsphere marked with the quality control antigen or antibody is 0.5-2 mg/ml, diluted at a ratio of 5%-20%, a dosage on the test strip is 0.005-0.1 µg/cm².

In one of the embodiments, the concentration of the quality control antigen or antibody marked with the fluorescent microsphere is 0.5 - 2 mg/ml, and a dosage is 0. 5-1.5 µg/cm; the concentration of the second BDNF monoclonal antibody coated on the detecting line is 0 .5~2 mg/ml, the dosage is 0.1-1 µg/cm.

In one of the embodiments, a distance between the marking line, the detecting line and the quality control line is 2-5 mm.

In one of the embodiments, a diameter of the fluorescent microsphere is 0.1 µm-1 µm, and an emission wavelength after being excited is 420 nm-800 nm.

Combined with Fig. 1, TX represents laser emission at 365nm, and RX represents signal collection at 615nm.

The present application uses fluorescent immunochromatography technology, by marking, spraying, coating and other processes, and by adjusting each process parameter one by one, the operation steps of BDNF detection are finally simplified, the reaction time is shortened, the detection reagents are stored and transported at room temperature, and the shelf life of the product is extended.

Compared with the prior art, the present application has the following significant advantages: short reaction time (completed in 15 minutes), simple operation steps (one-step reaction after adding a sample, no need for washing and multiple incubations), less equipment required (only one fluorescence analyzer is needed), the reagents are transported at room temperature (2-30°C), and the validity period is long (18 months).

### Embodiment 2

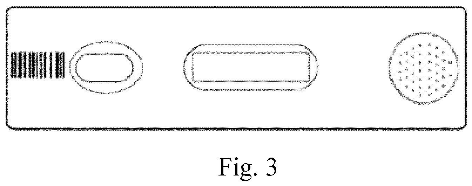

This embodiment provides a preparation method for a BDNF quantitative immunochromatography test strip, the preparation method is used to prepare the BDNF quantitative immunochromatography test strip of the above-mentioned embodiment 1, referring to Fig. 3, the preparation method specifically comprises:
Step 10: Overlap the water-absorbing pad 4 and the coating film on the base 1;

In this embodiment, the water-absorbing pad 4 and the coating film are overlapped on the base 1, one end of the water-absorbing pad 4 is close to the edge of the base 1, and one side of the coating film is overlapped by the water-absorbing pad 4 for 1-2 mm.

Step 20: Dilute fluorescent microspheres marked with first BDNF monoclonal antibody with marking buffer and dilute fluorescent microspheres marked with quality control antigen or antibody with marking buffer, mix the two marked microspheres, and spray on a binding pad to form a marker binding pad;

Wherein, the line sprayed on the bonding pad is the marking line 30.

Wherein, the concentration of the first BDNF monoclonal antibody marked with fluorescent microspheres is 0.5-2 mg/ml, and a dosage on the test strip is 0.03-0.3 µg/cm2; the concentration of the quality control antigen or antibody marked with the fluorescent microsphere is 0.5-2 mg/ml, a dosage on the test strip is 0.005-0.1 µg/cm²; the marking buffer comprises 0.2%~3% BSA, 5%-20% sucrose, 0.1%-0.5% Tween-20, 0.05-0.1% Proclin300, a balance of 0.01-0.05M, and PBS with a pH of 7.4.

Place the marker binding pad in an oven at 20-50°C with a humidity <30%, and dry it for 18-48 hours.

Wherein, the preparation step of the antibody marked with the fluorescent microsphere is as follows: after the fluorescent microspheres covalently activated by the conventional method are ultrasonically treated at 200W-400W for 20-30 seconds, add BDNF monoclonal antibody and quality control antigen/antibody according to the ratio of 50-200 µg marked antibody/100 µl fluorescent microspheres, after mixing, stirring and reacting at room temperature for 2 hours, centrifuging and washing 3 times, each time at 10000-16000xg, centrifuging for 30 minutes, dissolve the precipitate with PBS-T and ultrasonic treatment at 100W for 20 seconds, redissolve in PBS-T to the volume before centrifugation, and stored at 2-8°C. After the marker binding pad is prepared in advance, it is ready for step 40.

Step 30: Dilute the quality control antigen or antibody and second BDNF monoclonal antibody that can specifically bind to the antigen to be tested BDNF with coating buffer, and draw the diluted two raw materials in parallel in order to form a detecting line and a quality control line 31 on the coating film 32;

Wherein, the concentration of the quality control antibody or antigen coated on the quality control line is 0.5 - 2 mg/ml, and a dosage is 0.5-1.5 µg/cm; the concentration of the second BDNF monoclonal antibody coated on the detecting line is 0 .5~2 mg/ml, the dosage is 0.1-1 µg/cm, the coating buffer comprises 5% to 20% sucrose, a balance of 0.01-0.05M, and PBS with a pH of 7.4.

Place the coated plate coated with the quality control line 32 and the detecting line 31 in an oven at 20-50°C with a humidity <30%, dry for 18-48 hours, and store in a sealed and dry place at 20-30°C;

Step 40: Paste the marker binding pad and the sample pad 2 on the substrate 1 in an overlapping manner.

Specifically, the marker binding pad and the sample pad 2 are overlapped on the coating film, and the sample pad 2 is overlapped on the marker binding pad.

The present application uses fluorescent immunochromatography technology, by marking, spraying, coating and other processes, and by adjusting each process parameter one by one, the operation steps of BDNF detection are finally simplified, the reaction time is shortened, the detection reagents are stored and transported at room temperature, and the shelf life of the product is extended.

Compared with the prior art, the present application has the following significant advantages: short reaction time (completed in 15 minutes), simple operation steps (one-step reaction after adding a sample, no need for washing and multiple incubations), less equipment required (only one fluorescence analyzer is needed), the reagents are transported at room temperature (2-30°C), and the validity period is long (18 months).

### Embodiment 3

Based on the aforementioned embodiment 1, referring to Fig. 3, the present embodiment provides a test card, which comprises a test strip housing and a test strip, and the test strip consists of a sample pad, a marker binding pad (sprayed with fluorescent microspheres marked brain-derived nerve growth factor (BDNF) monoclonal antibody and protein for quality control), chromatographic membrane (test area (T) coated with BDNF monoclonal antibody, quality control area (C) coated with quality control protein antibody), water absorbing pad. Specifically, paste the chromatographic film and the water absorbing pad on the self-adhesive pad in order, and then use a gold sprayer to streak the BDNF antibody solution on the chromatographic film to dry and fix it. At the same time, the quality control protein antibody solution was streaked on the chromatographic film to dry and fix; Spray the solution containing BDNF and quality control protein on the marker pad (glass fiber) with a gold sprayer, dry and fix it, then cut and paste it on the self-adhesive pad; Cut the sample pad (including blocking agent, anti-RBC glass fiber) and paste it on the self-adhesive pad to obtain the detection plate. Cut the above-mentioned finally completed test plate into test strips with suitable width and put them into the test strip housing.

The detection card also comprises a parameter chip, which is an IC card or an ID card, and stores information such as a product calibration curve.

The method of using the test card is as follows:
a. When using a new batch of detection kits for the first time, the chip containing the parameters of this batch should be installed. Please refer to the requirements of the instrument manual for installation.
b. Open the packaging bag of the test card (the test card should be used immediately once opened).
c. Sample addition: Serum/Plasma: Take 100µL of serum or plasma sample, and drop it vertically to the sampling point of the test card; Whole blood: Take 100µL of whole blood sample, immediately add a drop of sample diluent to the sample point, and drop it vertically to the test card;
d. Instrument detection mode and external detection mode.

If the off-machine detection mode is used, the test card needs to be placed horizontally on the table. After adding the sample according to the method in c, the test card needs to stand outside the machine for 10 to 15 minutes, and then refer to the instrument manual for measurement.

If the in-machine detection mode is used, the test card needs to be placed on the test card tray of the analyzer. After adding the sample according to the sample adding method in c, operate according to the instrument manual, and the instrument will automatically start the test.
e. After the test is completed, the BDNF determination result of the sample will be displayed on the screen of the analyzer.

The above is only the implementation of the present application, and does not limit the protection scope of the present application. Any equivalent structure or equivalent process transformation made by using the contents of the specification and drawings of the present application, or directly or indirectly used in other related technical fields, is also included in the protection scope of the present application.

## Claims

1. A BDNF quantitative immunochromatographic test strip, comprising: a substrate and a coated film arranged on the substrate, wherein a sample pad, a coated line area and a water-absorbing pad are arranged on the coated film; the coated line area comprises a marking line, a detecting line and quality control line, wherein the marking line, the detecting line and the quality control line are arranged in parallel and separated from each other; the marking line is arranged close to the sample pad, the detecting line is arranged between the marking line and the quality control line, and the quality control line is arranged close to the water-absorbing pad;
wherein the marking line is the marking line coated with fluorescent microspheres marked with first BDNF monoclonal antibody and quality control antigen or antibody, the quality control line is the quality control line coated with quality control antibody or antigen, and the detection line is the detection line coated with second BDNF monoclonal antibody that can specifically bind to the antigen to be tested BDNF, and the first BDNF monoclonal antibody is different from the second BDNF monoclonal antibody.

2. The BDNF quantitative immunochromatographic test strip according to claim 1, wherein the antibody marked with fluorescent microspheres on the marking line is diluted with a marking buffer, and the marking buffer comprises 0.2%~3% BSA, 5%-20% sucrose, 0.1%-0.5% Tween-20, 0.05-0.1% Proclin300, a balance of 0.01-0.05M, and PBS with a pH of 7.4.

3. The BDNF quantitative immunochromatographic test strip according to claim 1, wherein the antibodies coated on the quality control line and the detection line are all diluted with a coating buffer, the coating buffer comprises 5% to 20% sucrose, a balance of 0.01-0.05M, and PBS with a pH of 7.4.

4. The BDNF quantitative immunochromatographic test strip according to claim 1, wherein the coating film is a nitrocellulose film, and the climbing speed of the nitrocellulose film is 95 s - 180 s/4 cm.

5. The BDNF quantitative immunochromatographic test strip according to claim 1, wherein the concentration of the first BDNF monoclonal antibody marked with fluorescent microspheres is 0.5-2 mg/ml, and a dosage on the test strip is 0.03-0.3 µg/cm2, diluted at a ratio of 10%-40%; the concentration of the quality control antigen or antibody marked with the fluorescent microsphere is 0.5-2 mg/ml, diluted at a ratio of 5%-20%, a dosage on the test strip is 0.005-0.1 µg/cm².

6. The BDNF quantitative immunochromatographic test strip according to claim 1, wherein the concentration of the quality control antibody or antigen coated on the quality control line is 0.5 - 2 mg/ml, and a dosage is 0.5-1.5 µg/cm; the concentration of the second BDNF monoclonal antibody coated on the detecting line is 0 .5~2 mg/ml, the dosage is 0.1-1 µg/cm.

7. The BDNF quantitative immunochromatographic test strip according to claim 1, wherein a distance between the marking line, the detecting line and the quality control line is 2-5 mm.

8. The BDNF quantitative immunochromatographic test strip according to claim 1, wherein a diameter of the fluorescent microsphere is 0.1 µm-1 µm, and an emission wavelength after being excited is 420 nm-800 nm.

9. A preparation method for a BDNF quantitative immunochromatography test strip, wherein the preparation method is used to prepare the BDNF quantitative immunochromatography test strip, and the preparation method comprises:
overlap a water-absorbing pad and a coating film on a substrate;
dilute fluorescent microspheres marked with first BDNF monoclonal antibody with marking buffer and dilute fluorescent microspheres marked with quality control antigen or antibody with marking buffer, mix the two marked microspheres, and spray on a binding pad to form a marker binding pad;
dilute the quality control antigen or antibody and second BDNF monoclonal antibody that can specifically bind to the antigen to be tested BDNF with coating buffer, and draw the diluted two raw materials in parallel in order to form a detecting line and a quality control line on the coating film;
paste the marker binding pad and the sample pad on the substrate in an overlapping manner.

10. The preparation method according to claim 9, wherein the overlap a water-absorbing pad and a coating film on a substrate comprising:
overlap the water-absorbing pad and the coated film on the substrate, and one end of the water-absorbing pad is close to the edge of the substrate, and one side of the coated film should be overlapped by the water-absorbing pad for 1-2 mm.
